# EUROPEAN PATENT SPECIFICATION

(11) **EP 0 507 193 B1**
(45) Date of publication and mention of the grant of the patent: **09.11.1994**
(21) Application number: 92105050.6
(22) Date of filing: 24.03.1992
(51) Int. Cl.: A61K 7/48, A61K 7/02

(54) **Oleaginous cosmetic containing collagen fiber powder**
Kollagenfaser-Pulver enthaltende ölhaltige kosmetische Zusammensetzung
Cosmétique oléagineux contenant de la poudre de fibres de collagène

(30) Priority: 01.04.1991 JP 68645/91; 04.02.1992 JP 19232/92
(43) Date of publication of application: 07.10.1992
(73) Proprietor: IDEMITSU PETROCHEMICAL CO. LTD., Tokyo 100 (JP)
(72) Inventor: Mohri, Kunihiko c/o Pola Chem. Ind. Inc., Shizuoka-ken (JP); Komachi, Nobuaki c/o Pola Chem. Ind. Inc., Yokohama-shi, Kanagawa-ken (JP); Nishi, Tohru c/o Idemitsu Petrochem. Co., Ltd., Himeji-shi, Hyogo-ken (JP); Kusamoto, Nobuo c/o Idemitsu Petrochem. Co., Ltd., Chuo-ku, Tokyo (JP)
(74) Representative: Dost, Wolfgang, Dr.rer.nat., Dipl.-Chem.

(56) References cited:
- EP-A- 0 036 199
- FR-A- 2 232 303
- GB-A- 2 216 797
- PATENT ABSTRACTS OF JAPAN vol. 10, no. 235 (C-366)(2291) 14 August 1986
- PATENT ABSTRACTS OF JAPAN vol. 11, no. 192 (C-429)(2639) 19 June 1987

## Description

### BACKGROUND OF THE INVENTION

### 1. FIELD OF THE INVENTION

This invention relates to oleaginous cosmetics, and particularly to an oleaginous makeup cosmetic.

### 2. DESCRIPTION OF THE RELATED ART

As oleaginous cosmetics, for example, lipstick, mascara, eye liner, foundation, sweat restraining stick and so on are generally known. Although these cosmetics which include pigment or powder are electrically neutral as a whole system, pigment or powder itself is known as electrifyable.

Electrification varies depending on the character of pigment or powder and oil components that are used together. For example, carbon black or titanium dioxide as a typical pigment is electrified positively in a nonpolar solvent, and negatively in a polar solvent. The oleaginous cosmetics which include electrified pigment or powder attract electrified substances as water vapor or dust in the air, clothes, metals or plastics existing outside the system although the cosmetics are electrically neutral as a whole.

As a specified example, when the cosmetic material is moved into a receptacle tank from a caldron in the process of production or when brushes are pulled out from a vessel in the use of eye liner or mascara the trouble is apt to occur that the cosmetic material attracts nearby opposite electric charges and scatters into the air to thereby stain nearby equipment or clothes.

After lipstick or foundation are applied to human faces, they are apt to adsorb dust in the air to thereby impair the effect of makeup sometimes.

In order to improve the problem, measures have conventionally been taken; i.e., an oleophilic surface active agent such as sorbitane fatty acid ester is added or the grounding of the equipment is reinforced or cosmetic vessels themselves are made hard to electrify.

However, the above-mentioned methods which have hitherto been known are not yet satisfactory because the pigment or powder included in the cosmetic can not be given enough resistance to electrification. Furthermore, addition of the oleophilic surface active agent would increase a sense of stickiness of the cosmetics when used, and stick-like cosmetics would reduce their hardness and deteriorate the quantity.

In view of the above, it is an object of the present invention to provide oleaginous cosmetics which have enough resistance to electrification as well as which has no problems with a sense of use or character of quality.

### SUMMARY OF THE INVENTION

As the result of research made by the present inventor to solve the above subjects, it was found that especially, a particular fine granulated powder which is obtained by grinding leather tissue or a collagen fiber is effective for the prevention of electrification of pigment and powder contained in the oleaginous cosmetics as well as it does not deteriorate a sense of use of or the quality of the cosmetic, and furthermore, it has an effect of giving a natural gloss or a sense of moistness. The present invention is completed on the basis of these facts.

The present invention relates to oleaginous cosmetics including a collagen fiber powder whereof the average grain size is 15 µm or less as well as the maximum grain size is 40 µm or less. Preferably, the oleaginous cosmetic includes a collagen fiber powder of 85 % or more by weight of cortex, 2 % or less by weight of oil and fat, and 3 % or less by weight, preferably 2 % or less by weight and more preferably 0.5 % or less by weight of a total quantity of Na⁺ and Ca²⁺ ions which can be extracted with water.

### DETAILED DESCRIPTION OF THE PREFERRED EMBODIMENT(S)

The present invention will be described hereinafter in detail.

The collagen fiber powder (hereinafter referred to as collagen powder) applied to the present invention is the powder which is obtained by grinding the collagen fiber obtained mainly from the leather and/or soft tissue of animals such as cattle, pigs, chickens.

The method of obtaining the collagen powder is described in detail, for example, in Japanese patent application Hei No.2-122001. The specified process in that the leather powder materials are ground coarsely, dried, degreased with the solvent, the remaining solvent is removed, and the resulting half-finished product is washed with water, dehydrated, swelled with steam, dried, and ground fine by a jet mill.

The average grain size of the collagen powder applied in the present invention is 15 µm or less, preferably, 7 µm or less and the maximum grain size is 40 µm or less, preferably, 15 µm or less.

Also, outside that range, the collagen powder has the effect of preventing electrification. However, in order to eliminate a sense of roughness and get a natural gloss, it is required that the average grain size of the powder is within the above range.

In order to improve a sense of quality as the cosmetics, it is required that it includes little impurities and preferably, that the cortex content is 85 % or more by weight.

If the fat and oil content which exists in the animal leather mingles in the cosmetic, it would be a cause of bad smell or coloring. Therefore, it is better that it includes less fat and oil, and preferably, 2 % or less by weight.

If there are many free ions which can be extracted with water among the impurities deriving from the leather materials, their salts (for example, NaCl, CaSO₄ etc.) will bleed out on the surface of the product, and as a result, impair the appearance of the article and a sense of use of the article. Therefore, it is better that an amount of ions which determines the amount of salt formed, i.e. the total amount of Na⁺ and Ca²⁺ ions, is less although small, i.e., 3 % or less by weight, more preferably 2 % or less by weight, and much more preferably 0.5 % or less by weight.

A cosmetic is provided which has a stable quality for a long time against sweat by using a collagen powder that satisfies these conditions.

Although the compounded amount of collagen powder is not restricted specially, it is enough to be within a range of 1-10 % by weight only for giving the effect of preventing electrification. Up to an amount of 50 % by weight can be compounded in conformity to a required sense of touch or a shape of the cosmetics.

Next, the oleaginous components which are used in the oleaginous cosmetic of the present invention are the same as the components hitherto compounded generally in the oleaginous cosmetics; for example, carnauba wax, beeswax, ceresin, liquid paraffin, squalene, solid paraffin, stearic acid, vatil alcohol, olive oil, vaseline, behenil alcohol, lanolin, isopropylmyristate, 2-ethylhexanoic acid triglycerol, and cetanol. The oleaginous component is compounded within a range of 20-98 % by weight, preferably, 25-90 % by weight against the whole oleaginous cosmetic.

Pigment or powder of the other cosmetic components except the collagen powder may be the ones applied to the oleaginous cosmetic generally in the past, and are compounded within a range of 0.1-40 % by weight against the whole oleaginous cosmetic.

In addition to the above-mentioned indispensable components, the oleaginous cosmetics of the present invention may include compounded humectants, various kinds of medicinal components, active agents, perfume or antiseptic. It goes without saying that these components are required to be applied under the condition of amounts that will not impair from the object of the present invention.

The present invention will be described in more detail with respect to an example of manufacturing the collagen powder as well as examples of cosmetics to which the collagen powder is applied.

First, the method of manufacturing the collagen powder will be described using an example of manufacturing the collagen powder.

The collagen powder was manufactured by the following process.

First, 1200 kg of a lump of chrome-tanned waste leather of cattle skin (shaving leather) was unraveled into the original shape of the shaving waste leather by a crusher, it was sent to a coarse grinding machine in sequence to make coarse ground leather powder of about 10 mm or less in grain size. The moisture of the coarse powder was 40-60 % by weight.

Next, 350 kg of the wet coarse ground leather powder was put into a vacuum drying machine and dried until the moisture became 20-30 % by weight. Subsequently, 270 kg of the dried coarse ground leather powder was degreased, and then filtrated. The content of the remaining fats and oils in the obtained degreased coarse powder was 0.5 % or less by weight.

Next, the solvent remaining in the degreased coarse leather powder was purged by steam until it did not smell.

Water at the ordinary temperature was supplied into the degreasing machine, and the leather powder was stirred for 30 minutes and drained by the filtration. This batch washing operation was performed four times in total to remove the free ions such as metal ions in the waste leather and water soluble components. The coarse ground leather powder contained 65-79 % by weight of moisture after the filtration and draining.

It was moved to a steam boiling machine without controlling humidity and stirred while boiled by a steam of 2 kg/cm²G at 130 °C for 45 minutes.

The boiled leather was dried preparatorily for 3 hours until the moisture contained 30-40 % by weight by a dryer kept at 90 °C. Thereafter, it was dried by a vacuum dryer at 45 °C for 8 hours to obtain 190 kg of the dried coarse ground leather whose moisture was 1 % or less by weight.

Next, the resulting leather was ground fine by a fine Victory mill. Subsequently, it was classified by a cyclonic classifier to get 35 kg of fine leather powder whose average grain size D₅₀ was about 30 µm and 155 kg of coarser leather powder whose average grain size D₅₀ was about 60 µm. The coarser leather powder was circulated in the above process of fine grinding.

Furthermore, 35 kg of this leather powder of D₅₀ = about 30 µm was again ground fine by a jet mill until the total amount of the powder had D₆₀ ≦ 7 µm.

Finally, it was classified by the cyclone to obtain 25 kg of collagen powder, which was handled as collagen powder A.

By the same process, the collagen powder B, C were obtained. The physical properties of each collagen powder are shown in Table 1.

**Table 1**

| | Collagen powder | | |
|---|---|---|---|
| | A | B | C |
| Average grain size (µm) | 5.7 | 6.1 | 6.5 |
| Maximum grain size (µm) | 10.1 | 12.7 | 12.7 |
| Cortex content (% by weight) | 89 | 94 | 92 |
| Fat & oil content(% by weight) | 0.4 | 0.3 | 0.3 |
| Amount of ions (% by weight)^{*} | 0.2 | 0.1 | 0.1 |

| | | | |
|---|---|---|---|
| ^{*}The total amount of Na⁺ and Ca²⁺ ions that can be extracted with water. | | | |

The present invention will be described hereinafter in more detail with respect to examples to which these collagen powders were compounded as well as controls to which oleophilic surface active agents conventionally used were compounded. The following compounding proportion is shown by weight part.

Collagen powder was compounded with an eye liner and the effect of the present invention was examined.

### (Manufacturing method)

A mixture mixed at the ratio shown in a section A of Table 2 was heated to be dissolved. The components shown in a section B were then added to and mixed with the dissolved mixture A and dispersed by three rolls to obtain an eye liner.

**Table 2**

| | | Example (part by weight) | Control (part by weight) |
|---|---|---|---|
| A | Microcrystalline wax | 10.0 | 10.0 |
| | Carnauba wax | 1.0 | 1.0 |
| | Dimethylsilicone | 61.0 | 61.0 |
| | Isopropylmyristate | 5.0 | 5.0 |
| | Sorbitanemonoisolate | - | 2.0 |
| B | Titanium dioxide | 5.0 | 5.0 |
| | Ultramarine | 15.5 | 15.0 |
| | Collagen powder A | 3.0 | - |

### (Evaluation)

An inventive product including the collagen powder was compared with a control product not including it in examination of electrifyability as follows.

A square nylon cloth sized 10 cm x 10 cm and electrified negatively was hung from a stand. A stainless microspatula to which about 0.1 g of eye liners of the inventive product and a control adhered was brought close to the cloth so as to be at a distance of 10 cm to the cloth to examine the scattered condition of the eye liner.

As a result, the eye liner of the present inventive product did not scatter at all while the control scattered greatly and stained the nylon cloth.

The effects of the collagen powders in the sweat restraining stick were compared.

### (Manufacturing method)

The components of a section A of Table 3 were mixed and heated to be dissolved. The components shown in a section B were added to and mixed with the former resulting mixture in the section A and was dispersed by a high speed disperser. The thus resulting mixture was poured into a component mold and caked by cooling to obtain a sweat restraining stick.

**Table 3**

| | | Example (part by weight) | Control (part by weight) |
|---|---|---|---|
| A | Solid paraffin | 8.0 | 8.0 |
| | Beeswax | 5.0 | 5.0 |
| | Liquid paraffine | 10.0 | 10.0 |
| | Glyceryl tri-isooctanate | 63.3 | 63.3 |
| | Glyceryl sesquiorate | - | 2.0 |
| B | Zinc oxide | 3.0 | 3.0 |
| | Aluminum chlorohydrate | 2.0 | 2.0 |
| | Cellulose powder | 5.0 | 5.0 |
| | Collagen powder B | 4.0 | - |

### (Evaluation)

An inventive product including the collagen powder and a control not including it were compared in electrifyability and organoleptic tests.

First, the results of the examination of electrifyability are shown as follows.

The sweat restraining sticks as the inventive product and the control one were left in the room whose humidity was 40% and whose temperature was 20 °C for a whole day and night to observe the amount of dust adhering to the surface of the sticks.

As a result, only slight dust adhered to the surface of the inventive stick whereas a great deal of dust adhered to the control stick. Most of the dust was chemical fibers electrified negatively.

Next, the result of the organoleptic test are shown as follows.

The sweat restraining sticks as the inventive product and the control one were used by 10 women aged from 18 to 20 at their depilated armpits. The organoleptic evaluation on a maximum scale of 5 points about a sense of stickiness and a sense of moistness were made. The average points for 10 women are shown in Table 4.

**Table 4**

| | Evaluation point of organoleptic test | |
|---|---|---|
| | Inventive product | Control product |
| Sense of sticki ness | 1.2 | 3.8 |
| Sense of moistness | 4.1 | 1.9 |

Furthermore, the effects of collagen powder in the lipsticks were compared.

### (Manufacturing process)

The respective components of a section A of Table 5 for the inventive and control products were mixed and heated to be dissolved. The mixtures of a section B were added and mixed with the corresponding mixtures of the section A thus obtained. The resulting semi-finished products were dispersed by three rolls, poured into corresponding molds and caked by cooling to obtain lipsticks.

**Table 5**

| | | Example (part by weight) | Control (part by weight) |
|---|---|---|---|
| A | Microcrystalline wax | 10.0 | 10.0 |
| | Carnauba wax | 2.0 | 2.0 |
| | Candelilla wax | 6.0 | 6.0 |
| | Lanoline | 20.0 | 20.0 |
| | Isopropylmyristate | 10.0 | 10.0 |
| | Castor oil | 46.0 | 46.0 |
| | Sorvitanemonoisostearate | - | 2.0 |
| B | Titanium dioxide | 2.0 | 2.0 |
| | Red No. 202 | 1.0 | 1.0 |
| | Collagen powder A | 3.0 | - |

### (Evaluation)

The inventive product including the collagen powder and the control one not including it were compared in hardness and organoleptic tests.

The measurement of hardness was made using a card tension meter at 25 °C. The inventive product was 200 g and the control one was 140 g in hardness, and as a result, a decrease in the hardness was improved.

The comparison by the organoleptic test was made by having 10 women aged from 18 to 22 put on their lips the lipsticks as the inventive product and the control one and evaluating on a maximum scale of 5 points a sense of roughness after the lipsticks were applied to their lips. The average point for 10 women is shown in Table 6.

**Table 6**

| | Evaluation points by organoleptic test | |
|---|---|---|
| | Inventive article | Control article |
| Sense of roughness | 2.6 | 4.1 |

According to the present invention, the resistance of the oleaginous cosmetic to electrification is greatly high as well as a sense of use or the quality of the cosmetic are not deteriorated as compared with the method hitherto known. Furthermore, the cosmetic brings about a natural gloss and a sense of moistness without a sense of roughness. Besides, a decrease in the hardness of a stick-like cosmetic is prevented.

## Claims

1. An oleaginous cosmetic comprising a collagen fiber powder having an average grain size of 15 µm and a maximum grain size of 40 µm.

2. An oleaginous cosmetic according claim 1, comprising pigment, powder and oil contents.

3. An oleaginous cosmetic according claim 2, wherein the proportion of the pigment and powder content in the whole oleaginous cosmetic is 0.1 - 40 % by weight.

4. An oleaginous cosmetic according claim 2, wherein the proportion of the oil content in the whole oleaginous cosmetic is 20 - 98 % by weight.

5. An oleaginous cosmetic according claim 2, wherein the proportion of the oil content in the whole oleaginous cosmetic is 25 - 90 % by weight.

6. An oleaginous cosmetic according claim 1, wherein the proportion of the collagen fiber content in the whole oleaginous cosmetic is 1 - 50 % by weight.

7. An oleaginous cosmetic according claim 1, wherein the proportion of the collagen fiber content in the whole oleaginous cosmetic is 1 - 10 % by weight.

8. An oleaginous cosmetic as claimed in claim 1, wherein the collagen fiber powder comprises 85 % or more by weight of a cortex, 2 % or less by weight of fat and oil and 3 % or less by weight of a total quantity of Na⁺ and Ca²⁺ ions which can be extracted with water.

9. An oleaginous cosmetic as claimed in claim 1, wherein the collagen fiber powder comprises 85 % or more by % by weight of a cortex, 2 % or less by weight of fat and oil and 0.5 % or less by weight of a total quantity of Na⁺ and Ca²⁺ ions which can be extracted with water.

10. An oleaginous cosmetic comprising a collagen fiber powder wherein the collagen fiber powder has an average grain size of 7 µm or less and a maximum grain size of 15 µm or less.

11. A method of making an oleaginous cosmetic by using an added collagen fiber powder, which is made by the steps of coarse grinding, drying, degreasing, solvent removing, water washing, dehydrating, steam swelling, drying, fine grinding, classifying between fine and coarse powders, and re-grinding the fine powders into an average grain size D₅₀ = 7 µm or less.

## Patentansprüche

1. Ölhaltige kosmetische Zusammensetzung, enthaltend ein Collagenfaser-Pulver mit einer mittleren Korngröße von 15 µm und einer maximalen Korngröße von 40 µm.

2. Ölhaltige kosmetische Zusammensetzung nach Anspruch 1, enthaltend Pigment-, Pulver und Ölbestandteile.

3. Ölhaltige kosmetische Zusammensetzung nach Anspruch 2, wobei der Anteil an Pigment- und Pulverbestandteilen in der gesamten ölhaltigen kosmetischen Zusammensetzung 0,1 bis 40 Gew.-% beträgt.

4. Ölhaltige kosmetische Zusammensetzung nach Anspruch 2, wobei der Anteil an Ölbestandteilen in der gesamten ölhaltigen kosmetischen Zusammensetzung 20 bis 98 Gew.-% beträgt.

5. Ölhaltige kosmetische Zusammensetzung nach Anspruch 2, wobei der Anteil an Ölbestandteilen in der gesamten ölhaltigen kosmetischen Zusammensetzung 25 bis 90 Gew.-% beträgt.

6. Ölhaltige kosmetische Zusammensetzung nach Anspruch 1, wobei der Anteil an Collagenfaser-Bestandteilen in der gesamten ölhaltigen kosmetischen Zusammensetzung 1 bis 50 Gew.-% beträgt.

7. Ölhaltige kosmetische Zusammensetzung nach Anspruch 1, wobei der Anteil an Collagenfaser-Bestandteilen in der gesamten ölhaltigen kosmetischen Zusammensetzung 1 bis 10 Gew.-% beträgt.

8. Ölhaltige kosmetische Zusammensetzung nach Anspruch 1, wobei das Collagenfaser-Pulver 85 Gew.-% oder mehr Cortex, 2 Gew.-% oder weniger Fett und Öl und 3 Gew.-% oder weniger einer Gesamtmenge an Na⁺ und Ca²⁺-Ionen enthält, die mit Wasser extrahiert werden können.

9. Ölhaltige kosmetische Zusammensetzung nach Anspruch 1, wobei das Collagenfaser-Pulver 85 Gew.-% oder mehr Cortex, 2 Gew.-% oder weniger Fett und Öl und 0,5 Gew.-% oder weniger einer Gesamtmenge an Na⁺ und Ca²⁺-Ionen enthält, die mit Wasser extrahiert werden können.

10. Collagenfaser-Pulver enthaltende ölhaltige kosmetische Zusammensetzung, wobei das Collagenfaser-Pulver eine mittlere Korngröße von 7 µm oder weniger und eine maximale Korngröße von 15 µm oder weniger besitzt.

11. Verfahren zur Herstellung einer ölhaltigen kosmetischen Zusammensetzung unter Verwendung eines Zusatzes an Collagenfaser-Pulver, umfassend folgende Schritte:
Grobmahlung, Trocknung, Entfettung, Losemittel-Entfernung, Wasserwäsche, Entwässerung, Dampfquellung, Trocknung, Feinmahlung, Klassierung in feines und grobes Pulver, und erneute Mahlung des feinen Pulvers zu einer mittleren Korngröße D₅₀ von 7 µm oder weniger.

## Revendications

1. Un cosmétique oléagineux comportant une poudre de fibre de collagène présentant une dimension moyenne de bain de 15 µm et une dimension maximale de bain de 40 µm.

2. Un cosmétique oléagineux selon la revendication 1, comportant des teneurs en pigment, en poudre et en huile.

3. Un cosmétique oléagineux selon la revendication 2, dans lequel la proportion de la teneur en pigment et en poudre dans l'ensemble du cosmétique oléagineux est de 0,1 à 40 % en poids.

4. Un cosmétique oléagineux selon la revendication 2, dans lequel la proportion de la teneur en huile dans l'ensemble du cosmétique oléagineux est de 20 à 98 % en poids.

5. Un cosmétique oléagineux selon la revendication 2, dans lequel la proportion de la teneur en huile dans l'ensemble du cosmétique oléagineux est de 25 à 90 % en poids.

6. Un cosmétique oléagineux selon la revendication 1, dans lequel la proportion de la teneur en fibre de collagène dans l'ensemble du cosmétique oléagineux est de 1 à 50 % en poids.

7. Un cosmétique oléagineux selon la revendication 1, dans lequel la proportion de la teneur en fibre de collagène dans l'ensemble du cosmétique oléagineux est de 1 à 10 % en poids.

8. Un cosmétique oléagineux selon la revendication 1, dans lequel la poudre de fibre de collagène comporte 85 % ou plus en poids d'une écorce, 2 % ou moins en poids de matière grasse et d'huile et 3 % ou moins en poids d'une quantité totale d'ions Na⁺ et Ca²⁺ pouvant être extraits par de l'eau.

9. Un cosmétique oléagineux selon la revendication 1, dans lequel la poudre de fibre de collagène comporte 85 % ou plus en poids d'une écorce, 2 % ou moins en poids de matière grasse et d'huile et 0,5 % ou moins en poids d'une quantité totale d'ions Na⁺ et Ca²⁺ pouvant être extraits par de l'eau.

10. Un cosmétique oléagineux comportant une poudre de fibre de collagène, dans lequel la poudre de fibre de collagène présente une dimension moyenne de grain de 7 µm ou moins et une dimension maximale de grain de 15 µm ou moins.

11. Un procédé pour fabriquer un cosmétique oléagineux par utilisation d'une poudre de fibre de collagène ajoutée, qui est fabriquée par les étapes de broyage grossier, séchage, dégraissage, élimination du solvant, lavage à l'eau, déshydratation, gonflement à la vapeur, séchage, broyage fin, classification entre poudres fines et poudres grossières, et rebroyage des poudres fines jusqu'à une dimension moyenne de grain D₅₀ = 7 µm ou moins.
